**Europäisches Patentamt**

(19) **European Patent Office**

**Office européen des brevets**

(11) Publication number: **0 136 035**
**B1**

(12) **EUROPEAN PATENT SPECIFICATION**

(45) Date of publication of patent specification: **13.09.89**

(51) Int. Cl.⁴: **C 12 N 15/00**

(21) Application number: **84305572.4**

(22) Date of filing: **16.08.84**

(54) **Associations of prokaryotic organisms and eukaryotic plant cells.**

(30) Priority: **18.08.83 GB 8322277**

(43) Date of publication of application:
**03.04.85 Bulletin 85/14**

(45) Publication of the grant of the patent:
**13.09.89 Bulletin 89/37**

(84) Designated Contracting States:
**AT BE CH DE FR GB IT LI NL SE**

(56) References cited:
**THE JOURNAL OF APPLIED BACTERIOLOGY,
vol. 56, no. 3, June 1983, pages 465-477,
Society for Applied Bacteriology by Blackwell
Scientific Publications; S.K.D. ALOYSIUS et al.:
"Artificially induced symbiotic associations of
L-form bacteria and plants"**

**THE JOURNAL OF APPLIED BACTERIOLOGY,
vol. 56, no. 3, June 1984, page 477, reference 1;
& ALOYSIUS S.K.D., 1982, "The induction of
non-pathogenic infections in plants with L-
phase bacteria", PhD Thesis, University of
Aberdeen**

(73) Proprietor: **The British Petroleum Company
p.l.c.
Britannic House Moor Lane
London EC2Y 9BU (GB)**

(72) Inventor: **Paton, Alan McEwan School of
Agriculture Building
University of Aberdeen 581 King Street
Aberdeen AB1 1UD Scotland (GB)**

(74) Representative: **Ryan, Edward Terrence et al
BP INTERNATIONAL LIMITED, Patents Division
Chertsey Road
Sunbury-on-Thames Middlesex, TW16 7LN (GB)**

Courier Press, Leamington Spa, England.

References cited:

JOURNAL OF APPLIED BACTERIOLOGY, vol.
49, 1980; S.K.D. ALOYSIUS et al.: "Induced
non-pathogenic infection of plant cells"

BIOLOGICAL ABSTRACTS RRM, Biosciences
Information Service, abstract no. 25029820,
Philadelphia, US; A.M. PATON et al.: "The
induction of nonpathogenic bacterial
infections in plants", & 13th International
Congress of Microbiology, Boston, August
8-13, 1982, page 79

BIOLOGICAL ABSTRACTS, vol. 58, Biological
Abstracts Inc., 1974, Philadelphia, US; S.M.
JONES et al.: "The L-phase of Erwinia
carotovora var. atroseptica and its possible
association with plant tissue", see pages
490-491, abstract no. 4593, & J. APPL.
BACTERIOL. 36(4), 729-737, 1973

AGRICULTURAL AND BIOLOGICAL
CHEMISTRY, vol. 45, no. 10, October 1981,
pages 2301-2309, T. YAMADA et al.:
"Polyethylene glycol-induced uptake of
bacteria into yeast protoplasts"

CHEMICAL ABSTRACTS, vol. 89, no. 3, July 17,
1978, page 187, abstract 18437v, Columbus,
Ohio, US; P.S. CARLSON: "Novel cellular
associations formed in vitro", & MOL. GENET.
MODIF. EUCARYOTES, (PROC. ANNU.
WORKSHOPS) 1974-1975 (PUB. 1977), 43-56

CHEMICAL ABSTRACTS, vol. 85, no. 19,
November 8, 1976, page 252, abstract 139683y,
Columbus, Ohio, US; K.L. GILES et al.: "Uptake
and continued metabolic activity of
Azotobacter within fungal protoplasts", &
SCIENCE 1976, 193(4258), 1125-6

## Description

The present invention relates to a method of introducing prokaryotic organisms into eukaryotic plant cells. This enables associations of eukaryotic plant cells and prokaryotic organisms to be produced in which the prokaryotic organisms remain within the eukaryotic plant cells without harming the cells.

There are various schemes for classifying living organisms. The scheme used for the purposes of the present specification is that given in on page 33 of "Elementary Microbiology" by O. Wyss, O. B. Williams and E. W. Gardner Jr., John Wiley & Sons Inc. 1963. In this classification the plant kingdom includes bacteria which are included in a phylum identified as the Protophyta (primitive plants without intracellular membranes). Eukaryotic plants therefore are all the members of the plant kingdom except the Protophyta.

It is known that cetain species of eukaryotic plants, for example angiosperms such as members of the order *Leguminosae,* can form symbiotic associations with certain prokaryotic organisms, namely certain species of bacteria, e.g. *Rhizobium* species. The bacteria, when in such an association, have the ability to take nitrogen from the air and convert it into combined forms which may be assimilated by the eukaryotic plant.

US 3 704 546 discloses a process which is alleged to produce artificially induced symbiotic associations of plant cells and bacteria, with the bacteria living within the plant cells. However the plant cells and bacteria disclosed are those for which symbiotic associations are known in nature. Examples are, the symbiotic associations between the *Leguminosae* and *Rhizobium* species which involve the bacteria entering and reproducing within nodules in the roots.

The ability to produce associations of eukaryotic plant cells and bacteria is potentially very valuable. Thus it might be possible to introduce bacteria having the ability to fix atmospheric nitrogen into plant species which do not form associations with such bacteria in nature.

A bacterium in its natural state is surrounded by a cell wall. It is known that bacteria associated with animals may be transformed into special forms known as L-forms (or L-phase) which do not have a cell wall. L-form bacteria are discussed in for example (a) Dienes, L. "Morphology and Reproductive Processes of Bacteria with Defective Cell Wall" and

(b) Wittler, R.G. "L-forms, Protoplasts, Spheroplasts—A survey of *in vitro* and *in vivo* studies" on (a) pp 74—93 and (b) pp 200—211 respectively of "Microbial Protoplasts, Spheroplasts and L-forms" edited by L.B. Guze, Williams and Wilkins Co., Baltimore, 1968.

The L-form bacteria may be produced by known methods which involve culturing the bacteria in a nutrient medium containing one or more agents which induce a transformation to the L-form. Some L-form bacteria are relatively stable i.e. they can be propagated in a nutrient medium without the continued presence of the inducing agents mentioned above. Other forms are unstable and revert to the normal form unless the presence of an inducing agent is maintained in the medium.

It has been suggested by Jones and Paton (1973) in the Journal of Applied Bacteriology, *36,* 729—737 that L-forms of plant pathogenic bacteria of the genus *Erwinia* may be able to enter plant cells and to produce within the plant cell. However if the L-form bacteria are not stable the bacteria may revert to the normal pathogenic state and any further reproduction of the bacteria in the plant cells will be harmful to the plant.

The formation of cultures of L-form bacteria is presently a time-consuming process. The formation, isolation and subsequent propagation of relatively stable L-form bacteria is even more time-consuming and uncertain.

According to one aspect of the present invention the process for introducing a prokaryotic organism into eukaryotic plant cells comprises bringing a suspension of bacteria in their normal state in a growth medium, which suspension is not toxic to the eukaryotic plant cell and which contains an effective amount of an L-form inducer, into contact with a eukaryotic plant cell.

By "bacteria in their normal state" we mean that the bacteria possess their usual cell walls.

The cells of eukaryotic organisms are not always directly accessible from outside the organism. Thus in leaf tissue a cuticle protects the cells in the leaf from the outside environment. A suspension of bacteria placed on the exterior of say a leaf will not be in contact with the walls of the plant cells. However a further embodiment of the present invention enables associations to be formed even when the portion of the eukaryotic organism to be treated is protected by a protective barrier such as a cuticle, or cutinized tissue, i.e. tissue which has a barrier layer having some of the characteristics of a cuticle.

In some cases plant tissue may have cell walls of greater than normal thickness, e.g. in callus tissue. The process of the present invention may still be used with such walls of increased thickness, although there may be some reduction in the extent of infection with L-forms. It is possible to prepare plant cells in the form of protoplasts without the normal cell wall, and these can be treated in accordance with the invention.

In the case of connected eukaryotic plant cell tissue the required contact between the suspension of bacteria mentioned above and the eukaryotic plant cell may be obtained by injecting the suspension into the tissue. This enables barrier layers such as cuticles to be by-passed.

By "connected tissue" we mean tissue in which the cells are in contact with each other as opposed to individual cells in suspension. Individual cells in suspension are found in suspension tissue culture.

Where the cell walls of the eukaryotic cells are directly accessible, simple contact between the suspension and the cell wall will be sufficient.

Thus the root of a complete plant, or a root cutting, containing root hairs or non-cutinized portions, may be immersed in the suspension. Alternatively the suspension may be applied to undifferentiated connected tissue e.g. a callus culture. Eukaryotic plant cells may be available as suspensions of cells either because in their natural state they are single cell plants or because they are artificially produced suspensions of the tissues of multicell plants. The prokaryotic cell may then be introduced by dispersing the eukaryotic plant cells in the bacterial suspension (either whole cells or protoplasts).

The aspects of the invention described above are concerned with forming associations from normal bacteria. It has been found that it is possible to introduce prokaryotic organisms into eukaryotic plant cells without requiring direct contact between bacteria in their normal state and eukaryotic plant cells.

According to a further aspect of the present invention there is provided a process for using an association of prokaryotic organisms and eukaryotic plant cells which comprises macerating eukaryotic plant cell tissue infected with L-form bacteria, and bringing a liquid released by maceration of the cells into contact with a eukaryotic plant cell.

The liquid may be used as it is, without the addition of nutrients or L-form inducers.

It has further been found that the absence of bacteria in their normal state can be assured by the use of a bacterial filter but that nevertheless material capable of passing through a bacterial filter can be used to introduce prokaryotic organisms into eukaryotic cells.

According to a further embodiment of the present invention a prokaryotic organism is introduced into a eukaryotic cell by forming an aqueous suspension of L-form bacteria, passing the suspension through a bacterial filter and bringing the liquid filtrate into contact with the cell wall of a eukaryotic cell.

By "bacterial filter" we mean a filter with a pore size such that bactria are unable to pass through the filter. Examples of suitable filters are those having a pore size of 0.8 μm, or even filters having a pore size of 0.22 μm. The ability to use liquid which has been passed through such a filter means that undesirable bacteria can be excluded.

The suspension of L-form bacteria subjected to filtration may for example be obtained by maceration of eukaryotic plant cell tissue infected with L-form bacteria.

The eukaryotic cells used in the various aspects of the present invention may be from the seed plants (angiosperms, gymnosperms), fungi, algae or yeasts. Examples of angiosperms which may be used are monocotyledons e.g. *Graminaceae*, and dicotyledons e.g. *Leguminosae, Solanaceae.* Among specific examples of plants which may be used are bean, clover, lucerne, maize and *Arabidopsis.*

The bacteria which may be used may be filamentous or non-filamentous and may be pathogenic or nonpathogenic bacteria. By nonpathogenic bacteria we mean bacteria which do not in their normal state have the ability to invade plant tissue so as to cause plant diseases.

It should be noted that although the normal form of a pathogenic bacterium is harmful to a given eukaryotic plant the plant appears to be able to tolerate the L-form without producing disease symptoms.

The bacterium may be Gram negative e.g. *Pseudomonas, Azotobacter, Bejerinckia*, or Gram-positive eg *Bacillus.* Examples of filamentous bacteria which may be used are *Streptomyces.*

The bacteria are suspended in a growth medium when used in their normal form. Growth media for bacteria are well known. The growth medium must not of course contain material which will kill the sample of connected plant tissue or cell into which the mixture of bacteria and growth medium is brought into contact. The toxicity of any proposed growth medium may be determined by simple tests.

Materials for inducing the production of L-form bacteria are disclosed in the literature. Examples of L-form inducers which may be used include penicillins and lysozyme. Mixtures of L-form inducers may be used.

It is desirable for the concentration of bacteria in the growth medium to be relatively high e.g. greater than $10^4$ cells/ml, more preferably greater than $10^7$ cells/ml.

The amount of mixture which is injected into the plant tissue may be relatively small for example no more than 0.5 ml.

The invention will now be further illustrated by reference to the following examples.

Example 1

A fresh suspension of a young culture of *Azotobacter* sp. (grown on a nutrient medium for 24h to 48h at 25°C) was prepared in liquid medium "B" so as to provide a distinctly cloudy appearance (containing $10^7$ to $10^8$ bacterial cells per millilitre).

Liquid "B" medium was prepared from a concentrated liquid medium "A" by adding aseptically 1 part "A" to 9 parts sterile isotonic saline (0.85% w/v NaCl). The medium was completed by the aseptic addition of 10% inactivated horse serum.

Liquid medium "A" consisted of sucrose (20.0 g), glucose (0.5 g), peptone (0.5 g), magnesium sulphate (0.01 g), yeast extract (0.5 g), distilled water (100 ml). After sterilisation by autoclaving (15 psi, 121°C, 15 min.) benzylpenicillin (to a final concentration of 10,000 i.u./ml) was added aseptically at not greater than 50°C.

Both liquids were stored at 2°C and used within 7 days of preparation.

The suspension of bacteria in medium "B" was incubated for 12 to 24 hours at 27°C before being used as a plant inoculum.

Aliquots of the bacterial suspensions as thus prepared were slowly injected by hypodermic syringe into healthy plant tissue. The volume of suspension injected varied with the nature of the plant tissue (for example 0.5 ml per injection was used for inoculating cucumber fruit). The injected plant tissue was incubated either at room temperature or at 25°C for 48h prior to examination.

For examination the tissue was subjected to enzymic macerating activity and the resultant separated plant cells were observed by interference and phase contrast microscopy. Confirmation of the presence of the intracellular L-form bacteria was made using the immunofluorescence technique with anti-*Azotobacter* serum.

The above described technique was applied to plant tissue from bean (*Phaseolus*), clover (*Trifolium pratense*), lucerne (alfalfa), maize, *Arabidopsis*, and cucumber.

Example 2

Using a similar suspension of an *Azotobacter* sp. in the same Medium ("B") as in Example 1 associations were produced in germinating seeds which continued in the developing seedlings and plants.

Seeds of both monocotyledons (maize) and dicotyledons (bean) were surface sterilised by hypochlorite treatment, washed, and immersed during their germination period in the bacterial suspension described above. A suitable procedure was to germinate the sterilised seed on a sterile water agar surface. Each seed was then surface inoculated with a few drops of the bacterial suspension and incubated at ambient temperatures or at 25°C until germination was complete.

Confirmation of the presence of intracellular L-forms was made as in Example 1.

Example 3

Cultured plant tissue (soya bean), either as callus or as cell suspensions, were associated with an *Azotobacter* sp.

(a) The callus sited on an agar substrate was flooded with the bacterial suspension in the same Medium B as in Example 1 and thereafter the material was incubated for a period of 24 to 48 hours at 25°C. Thereafter the callus was washed in sterile saline and replaced on a suitable sterile culture medium and allowed to develop.

(b) Suspensions

Suspension cultures of soya bean cells were washed and the plant tissue culture medium replaced with the bacterial suspension in Medium B. The mixture was incubated and aerated by rotation for 24 hours at 25°C. Thereafter the liquid Medium "B" was replaced by the liquid plant tissue culture medium and the plant cells were cultured as before the treatment.

The presence of intracellular L-forms was confirmed in the same manner as before.

Example 4

Cucumber tissue infected by the manner described in Example 1 was macerated by a mechanical blender. The roots of seedling plants, eg *Brassicae,* were immersed in a shallow layer of the macerate for 24 hours at 27°C and after washing in tap water were allowed to develop for a further 24 to 48 hours on the surface of the water agar. The root hairs and root cortical cells were found to be infected with large numbers of L-forms.

Example 5

When the macerate described in Example 4 was pre-filtered to remove coarse debris and finally filtered through membranes of pore size 0.8 μm or 0.22 μm the filtrate was capable of infecting root hairs and cortical cells of plants with L-forms to a similar degree as with the crude macerate. When re-inoculated into cucumber tissue the membrane filtrate produced L-form infections of the constituent cells.

Example 6

A tobacco seedling (*Nicotiana* spp.) was inoculated with a culture of L-form *Azotobacter* prepared as described above. L-form bacteria were subsequently identified in the pith of the mature plant and in the callus tissue prepared from the pith of a secondary shoot. After subculturing using standard techniques and culture media through many generations, L-form bacteria could still be detected in th callus tissue. When some of this callus differentiated to form a new plant and this plant was grown on L-form *Azotobacter* could be identified within this plant (ie more than one year after the initial injection).

Example 7

It is an important feature of the invention that original metabolic functions of the prokaryotic cells may continue while such cells are incorporated as L-forms within eukaryotic cells.

An effect of the prokaryotic/eukaryotic associations was demostarated by immersing the roots of sterile yound seedlings of radish (Raphianus) in either (a) a non-filtered macerate of cucumber fruit previously infected with a nisin (antibiotic) producing strain of the bacterium S. lactis by the method described in Example 1 or, (b) a membrane (pore size 0.22 μ) filtrate of (a).

The seedlings were allowed to develop for 48h on the surface of sterile water agar and were thereafter planted in trays of non-sterile seedling compost.

Untreated seedlings of the same age were similarly planted as controls.

The seedlings were allowed to deveolop in normal plant growth conditions for 7 days. Filter-sterilised extracts from the crushed leaves and stems of the plants treated by both methods (a and b) showed inhibiting activity against a nisin-sensitive strain of the bacterium *Microcollus luteus*. No inhibitions was observed with extracts from the untreated plant tissues.

Similar results were obtained using the L-forms of bacteria producing antibiotics such as subtilin, polymyxin and bacitractin.

Example 8

Protoplasts of *Arabidopsis thaliani* were prepared by enzymic cell-wall degradation in suitable osmotic conditions. A suspension of such protoplasts were mixed with a suspension of *Azotobacter* in Medium B (see Example 1). The mixture was incubated with mild aeration for 48h at 25°C. Thereafter on examination by interference microscopy a high proportion of the plant cells was found to contain L-forms. The identification of the inclusions was confirmed by the immunofluorescence technique.

## Claims

1. A process for introducing prokaryotic organisms into eukaryotic plant cells which comprises bringing a suspension of bacteria in their normal state in a grow medium, which suspension is not toxic to the eukaryotic cell and which contains an effective amount of an L-form inducer into contact with a eukaryotic plant cell.

2. A process according to claim 1 which comprises injecting connected eukaryotic cell tissue with a suspension of normal bacteria in a growth medium which is not toxic to the eukaryotic cell and which contains an effective amount of an L-form inducer.

3. A process for introducing prokaryotic organisms into eukaryotic cells which comprises macerating eukaryotic cell tissue infected with L-form bacteria, and bringing a liquid released by maceration of the cells into contact with the cell wall of a eukaryotic cell.

4. A process for the introduction of a prokaryotic organism into a eukaryotic cell which comprises forming an aqueous suspension of L-form bacteria, passing the suspension through a bacterial filter, and bringing the liquid filtrate into contact with the cell wall of a eukaryotic cell.

5. A process according to any one of the preceding claims wherein the plant cells are in the form of a tissue culture.

6. A process according to any one of the preceding claims wherein the eukaryotic plant cell is a cell from an angiosperm.

7. A process according to claim 6 wherein the cell is a cell of a monocotyledonous plant.

8. A process according to claim 7 wherein the cell is cell of a plant from the Graminaceae.

9. A process according to any one of claims 1 to 6 wherein the cell is a cell of a dicotyledonous plant.

10. A process according to any one of the preceding claims wherein the cell is a cell of a plant from the Leguminosae.

11. A process according to claims 1 or 2 wherein the concentration of bacteria in the growth medium is greater than $10^4$ cells/ml.

12. A process according to any one of the preceding claims wherein the eukaryotic plant cell is in the form of protoplast.

## Patentansprüche

1. Verfahren zum Einbringen von prokaryotischen Organismen in eukaryotische Pflanzenzellen, welches Verfahren das Einbringen einer Suspension von Bakterien in ihrem normalen Zustand in ein Wachstumsmedium, welche Suspension nicht toxisch gegenüber den eukaryotischen Zellen ist und eine wirksame Menge eines L-Form-Induktors enthält, und das in Berührung Bringen der Suspension mit einer eukaryotischen Pflanzenzelle umfaßt.

2. Verfahren nach Anspruch 1, welches das Injizieren einer Suspension von normalen Bakterien in einem Wachstumsmedium, das nicht toxisch gegenüber den eukaryotischen Zellen ist und eine wirksame Menge eines L-Form-Induktors enthält, in zusammenhängendes eukaryotisches Zellgewebe umfaßt.

3. Verfahren zum Einbringen von prokaryotischen Organismen in eukaryotische Zellen, welches das Mazerieren von mit L-form-Bakterien infiziertem eukaryotischem Zellgewebe und das in Berührung Bringen einer Flüssigkeit, die durch Mazeration der Zellen freigesetzt wird, mit der Zellwand einer eukaryotischen Zelle umfaßt.

4. Verfahren zum Einbringen eines prokaryotischen Organismus in eine eukaryotische Zelle, welches das Bilden einer wässerigen Suspension von L-Form-Bakterien, Leiten der Suspension durch einen Bakterienfilter und das in Berühung Bringen des flüssigen Filtrates mit der Zellwand einer eukaryotischen Zelle umfaßt.

5. Verfahren nach einem der vorhergehenden Ansprüche, worin die Pflanzenzellen die Form einer Gewebekultur aufweisen.

6. Verfahren nach einem der vorhergehenden Ansprüche, worin die eukaryotische Pflanzenzelle eine Zelle von einer Angiosperme ist.

7. Verfahren nach Anspruch 6, worin die Zelle eine Zelle von einer einkeimblättrigen Pflanze ist.

8. Verfahren nach Anspruch 7, worin die Zelle eine Zelle von einer Pflanze der Gattung der Graminaceen ist.

9. Verfahren nach einem der Ansprüche 1 bis 6, worin die Zelle eine Zelle von einer zweikeimblättrigen Pflanze ist.

10. Verfahren nach einem der vorhergehenden Ansprüche, worin die Zelle eine Zelle von einer Pflanze der Gattung der Leguminosen ist.

11. Verfahren nach Anspruch 1 oder 2, worin die Konzentration an Bakterien in Wachstumsmedium größer als $10^4$ Zellen/ml ist.

12. Verfahren nach einem der vorhergehenden Ansprüche, worin die eukaryotische Pflanzenzelle die Form eines Protoplasten aufweist.

## Revendications

1. Procédé pour introduire des organismes procaryotes dans des cellules végétales , ou cellules de plantes, eucaryotes, qui comprend la mise en contact d'une suspension de bactéries, à leur état normal, dans un milieu de croissance (suspension qui n'est pas toxique pour la cellule eucaryote et qui contient une quantité efficace d'un inducteur de forme L), avec une cellule de plante, ou cellule végétale eucaryote.

2. Procédé selon la revendication 1, qui comprend l'injection, à du tissu connecté de cellules eucaryotes, d'une suspension de bactéries normales dans un milieu de croissance qui n'est pas toxique pour la cellule eucaryote et qui contient une quantité efficace d'un inducteur de la forme L.

3. Procédé pour introduire des organismes procaryotes dans des cellules eucaryotes, qui comprend la mise en macération d'un tissu de cellules eucaryotes infecté par des bactéries de forme L, et la mise en contact d'un liquide, dégagé par la macération des cellules, avec la paroi cellulaire d'une cellule eucaryote.

4. Procédé pour l'introduction d'un organisme procaryote dans une cellule eucaryote, qui comprend la formation d'un suspension aqueuse de bactéries de forme L, le passage de la suspension à travers un filtre bactérien (filtre de retenue de bactéries) et la mise du filtrat liquide en contact avec la paroi cellulaire d'une cellule eucaryote.

5. Procédé selon l'une quelconque des revendications précédentes, dans lequel les cellules végétales sont sous la forme d'une culture de tissu.

6. Procédé selon l'une quelconque des revendications précédentes, dans lequel la cellule végétale eucaryote est une cellule provenant d'un angiosperme.

7. Procédé selon la revendication 6, dans lequel la cellule est une cellule d'une plante monocotylédone.

8. Procédé selon la revendication 7, dans lequel la cellule est une cellule d'une plante provenant des graminées.

9. Procédé selon l'une quelconque des revendications 1 à 6, dans lequel la cellule est une cellule d'une plante dicotylédone.

10. Procédé selon l'une quelconque des revendications précédentes, dans lequel la cellule est une cellule d'une plante provenant des légumineuses.

11. Procédé selon la revendication 1 ou 2, dans lequel la concentration des bactéries dans le milieu de croissance est supérieure à $10^4$ cellule/ml.

12. Procédé selon l'une quelconque des revendications précédentes, dans lequel la cellule végétale eucaryote est sous la forme d'un protoplaste.